# FASCICULE DE BREVET EUROPEEN

(11) **EP 2 972 358 B1**
(45) Date de publication et mention de la délivrance du brevet: **01.11.2017**
(21) Numéro de dépôt: 14715356.3
(22) Date de dépôt: 11.03.2014
(51) Int. Cl.: G01N 33/537, G01N 33/558, G01N 33/543, G01N 33/569

(54) **NOUVELLE MÉTHODE DE DOSAGE EN FLUX D'UN OBJET D'INTÉRÊT**
NEUARTIGES FLUSSASSAYVERFAHREN FÜR EIN OBJEKT VON INTERESSE
NOVEL FLOW ASSAY METHOD FOR AN OBJECT OF INTEREST

(30) Priorité: 14.03.2013 FR 1352298
(43) Date de publication de la demande: 20.01.2016
(73) Titulaire: Horiba ABX SAS, 34184 Montpellier cedex 4 (FR); Bibette, Jérôme, 75005 Paris (FR)
(72) Inventeur: DAYNES, Aurélien, 34090 Montpellier (FR); CAUET, Gilles, 34270 Fontanes (FR); GINEYS, Jean-Philippe, 30440 Roquedur (FR); NERIN, Philippe, 34820 Assas (FR); BIBETTE, Jérôme, 75005 Paris (FR)
(74) Mandataire: Santarelli
(86) Numéro de dépôt international: PCT/FR2014/050544
(87) Numéro de publication internationale: WO 2014/140468

(56) Documents cités:
- EP-A2- 0 056 018
- WO-A2-00/29617
- FR-A5- 2 158 940
- US-A- 4 879 211
- GAO H L ET AL: "Amperometric Immunosensor Based on Magnetic Inorganic Bionanoparticles Sensing Films", CHINESE JOURNAL OF ANALYTICAL CHEMISTRY,, vol. 36, no. 12, 1 décembre 2008 (2008-12-01), pages 1614-1618, XP025769481, ISSN: 1872-2040, DOI: 10.1016/S1872-2040(09)60008-6 [extrait le 2008-12-01]

## Description

La présente invention concerne une méthode de dosage en flux dans un milieu liquide d'un objet (ou élément) d'intérêt par la formation d'agrégats de particules fonctionnalisées en surface par au moins une molécule fonctionnalisante, par ailleurs appelées dans le texte récepteur, spécifique dudit objet d'intérêts. L'invention est seulement limités par les revendications. Selon l'invention, par objet d'intérêt il est entendu toute substance sous réserve qu'elle puisse être reconnue spécifiquement par au moins deux récepteurs. Comme objet d'intérêt on peut citer, à titre d'exemple et sans limitation, tout antigène pouvant réagir avec un anticorps ou un aptamère, toute molécule d'acide nucléique pouvant être reconnue par une molécule d'acide nucléique complémentaire, toute cellule ou fragment de cellules ou tout microorganisme ou fragment de microorganisme ou encore toute molécule chimique, pour peu qu'on leur connaisse un récepteur spécifique. Ainsi il apparaît possible d'utiliser la méthode selon l'invention pour quantifier des protéines, des anticorps, des acides nucléiques, des cellules, des fragments de cellule, des microorganismes (par exemple bactéries, certains champignons, algues vertes ou encore virus), des fragments de microorganisme, ou encore des molécules chimiques.

Dans la plupart des applications de laboratoire, le dosage d'un composé biochimique au sein d'un fluide biologique fait appel à une réaction visant à établir des liens entre une substance recherchée et un récepteur qui lui est spécifique. Ce récepteur peut par exemple être un anticorps ou un aptamère dans le cadre de la détection d'une protéine, ou une séquence d'acide nucléique dans le cadre de la détection d'acide nucléique complémentaire.

Un système d'immuno-diagnostic très utilisé dans l'art antérieur repose sur la fonctionnalisation de micro ou nano particules par des récepteurs, aptes à reconnaitre et à se lier à l'objet d'intérêt recherché permettant, en présence dudit objet d'intérêt recherché, la formation de complexes particulaires formés d'au moins une particule fonctionnalisée liée audit objet d'intérêt recherché par ledit récepteur ou d'agrégat particulaire comprenant au moins deux particules fonctionnalisées, liées entre elles par un objet d'intérêt.

Par la suite, la détection des complexes ou des agrégats particulaires formés est souvent réalisée par une mesure optique. La sensibilité est quant à elle bien souvent liée au principe de détection utilisée et à l'instrumentation associée.

Par exemple, lorsque l'objet d'intérêt recherché est un antigène présent dans un milieu donné, celui-ci peut présenter plusieurs épitopes. On peut alors fixer chimiquement à la surface des particules utilisées des anticorps dirigés contre ledit antigène pour les fonctionnaliser. Lorsque lesdites particules fonctionnalisées sont introduites dans le milieu, une réaction peut avoir lieu entre lesdites particules fonctionnalisées et ledit antigène. Ainsi une première particule fonctionnalisée peut capter l'antigène recherché et former un premier complexe particulaire. Mais bien entendu, du fait du mouvement brownien, ce même antigène fixé à une première particule fonctionnalisée peut capter une seconde particule elle-même fonctionnalisée. Les deux particules fonctionnalisées se trouvent donc liées via la formation de liaisons anticorps-antigène-anticorps et constituent donc un agrégat. La réaction peut se poursuivre pour ainsi former des agrégats de plus grande taille réunissant un grand nombre de particules liées entre elles selon le même principe.

La présence d'agrégats au sein dudit milieu augmente la diffusion de lumière, de sorte que l'intensité d'un faisceau de lumière traversant ledit milieu est diminuée. C'est le principe de la turbidimétrie optique dont l'avantage principal est la simplicité de mise en oeuvre, permettant d'envisager la fabrication de dispositifs à prix compétitif.

Cependant, les performances d'un tel système sont relativement limitées. En effet, pour permettre la formation de liens dans un délai raisonnable, la concentration en particules doit être importante. De plus, les particules qui ne réagissent pas (particules introduites restées libres) ne contribuent pas au signal utile. Par contre par leur capacité à diffuser la lumière, elles contribuent à la formation d'un bruit de fond qui peut s'avérer gênant pour la mesure.

Afin de diminuer la quantité en particules introduites restées libres, des techniques ont été mises au point pour augmenter la fréquence des collisions entre particules. Ces méthodes peuvent employer des champs magnétiques [Baudry J. et al., PNAS, 103 (2006) 16076-16078] ou électriques [Iwata K. et al., Annals of Chemical Biochemistry, 46 (2009) 117-122], ou des ultrasons [Wiklund M., Hertz H.M., Lab on a Chip, 6 (2006), 1279-1292] pour augmenter localement la concentration en particules et favoriser la formation d'agrégats particulaires. WO0029617 décrit l'utilisation des points quantiques pour une détection ultrasensible. Cependant, même dans ce cas, d'autres problèmes peuvent être rencontrés. Par exemple une concentration en objet d'intérêt inférieure au picomolaire (pM) ne peut généralement pas être détectée, du fait notamment des fluctuations thermiques du milieu qui entraînent des fluctuations du signal optique.

La détection et le dosage d'objets d'intérêt, particulièrement biologiques, dans un échantillon, avantageusement biologique, par mesures en flux sont connus de l'homme de l'art. Toutes les méthodes de l'art antérieur utilisent des microparticules dont le niveau de dispersion est suffisamment faible pour permettre leur identification dans un espace de représentation bi-paramétrique. Par exemple, la représentation bi-paramétrique diffusion aux petits angles (Forward Scattering, FSC) x diffusion aux grands angles (Side Scattering, SSC) permet d'identifier la présence d'agrégats colloïdaux de tous ordres, et le traitement des représentations bi-paramétriques permet de connaître le nombre et la nature de ces agrégats. Cependant lorsque des agrégats de plus de deux particules sont formés, même l'utilisation de particules monodisperses n'empêche pas les populations d'agrégats de se chevaucher.

Généralement, la concentration en objet d'intérêt est donc déterminée en calculant le rapport entre le nombre d'agrégats formés et le nombre de particules isolées, sans tenir compte de la multiplicité des agrégats. Aux fortes concentrations en objet d'intérêt, les agrégats peuvent être constitués de nombreuses particules. Malheureusement quelle que soit la taille des agrégats dans les méthodes de quantification de l'art antérieur ces agrégats bien que pris en compte ne sont pas pondérés par le nombre de liens formés. ils seront alors considérés de la même importance qu'un doublet défini comme un agrégat formé par deux particules liées entre elles par un objet d'intérêt, alors qu'ils représentent un état d'agrégation plus important. Ainsi un nombre non négligeable de liens créés par l'objet d'intérêt sera négligé, et la précision de la mesure s'en trouvera dégradée aux fortes concentrations en objets d'intérêt.

De plus dans l'art antérieur, pour une détection en flux, les particules doivent posséder un niveau de qualité suffisant pour permettre leur détection pour le dosage précis de l'objet d'intérêt recherché. Idéalement, les particules doivent être identiques, notamment par leur volume, puisque deux particules prises aléatoirement dans le milieu réactionnel doivent conduire à former un agrégat dont la section efficace doit être sensiblement invariable. En général, les particules utilisées pourront être des sphères dont les diamètres pourront être distribués aléatoirement autour d'une valeur moyenne avec un certain écart type caractéristique de la qualité du fractionnement des particules. On estime alors que le coefficient de variation de la taille des particules pourra être inférieur ou égal à 10 %. Ainsi, dans l'art antérieur la mise en oeuvre d'un test biologique utilisant des mesures en flux nécessite des particules de qualités suffisantes, dont la dispersion de taille doit être suffisamment maîtrisée et reproductible.

Ceci constitue une contrainte industrielle forte puisque des coefficients de variation inférieurs à 10 % nécessitent des soins de fabrication importants conduisant à des prix de revient des particules, et par conséquent de la mesure, plus importants. De plus, une bonne maîtrise du coefficient de variation de la taille des particules est difficile à atteindre notamment lorsque l'on cherche à donner aux particules des propriétés physiques combinant notamment des propriétés physiques spécifiques telles que la fluorescence ou le magnétisme.

Or, de telles propriétés peuvent être souhaitables pour procéder à une accélération de la réaction d'agrégation.

D'autres techniques de détection des objets d'intérêt utilisant la détection de particules en flux existent. On peut citer la technologie Luminex qui détecte des antigènes grâce à des particules micrométriques intrinsèquement fluorescentes (fluorescence primaire) recouvertes d'anticorps spécifiques dudit antigène. Une fois ledit antigène capturé par lesdits anticorps spécifiques recouvrant les particules, il est marqué secondairement par un second anticorps, lui-même fluorescent (fluorescence secondaire) à une longueur d'onde différente de celle de la fluorescence primaire desdites particules. La fluorescence secondaire sert à la détection des s complexes anticorps secondaires-antigène. Mais on comprend aisément que cette méthode requiert plusieurs étapes d'une part d'incubation puis d'autre part de lavage pour éliminer les réactifs en excès qui pourraient interférer avec la mesure. Elle est donc plus longue et complexe à mettre en oeuvre qu'un test d'agrégation qui ne demande qu'une seule étape.

Après de nombreuses recherches, les inventeurs ont mis au point une méthode de quantification d'un objet d'intérêt donné, basée sur la détermination de l'état d'agrégation d'une suspension de particules fonctionnalisées mises en contact avec un objet d'intérêt donné tout en surmontant les problèmes de l'art antérieur. En particulier la méthode selon l'invention pourra s'appliquer à des ensembles de particules fonctionnalisées qui présentent une dispersion de taille importante, ce qui n'est pas le cas des techniques de l'art antérieur.

L'invention vise donc à proposer une nouvelle méthode de quantification d'un objet d'intérêt donné préalablement prélevé, par mesure en flux des agrégats formés au cours d'une réaction objets d'intérêt recherché / particule fonctionnalisée, par une méthode qui surmonte tout ou partie des inconvénients des méthodes connues de l'art antérieur.

Selon l'invention, par agrégat on entend un agrégat particulaire comprenant au moins deux particules fonctionnalisées, liées entre elles par un objet d'intérêt (doublet). On comprend donc que selon l'invention on nomme "agrégat" tout agrégat particulaire qu'il soit formé de deux particules fonctionnalisées liées entre elles par un objet d'intérêt ou de plus de deux particules fonctionnalisées liées entre elles par plusieurs objets d'intérêt. On comprend aussi que les particules fonctionnalisées liées à un seul objet d'intérêt sont considérées comme des singulets.

Donc par singulet on entend dans le présent texte aussi bien les particules fonctionnalisées libres de tout lien et les objets d'intérêt liés à une seule particule fonctionnalisée.

La méthode selon l'invention est donc assimilable à un test d'agrégation (ou encore d'agglutination).

Le signal utile dans un test d'agrégation est le nombre de liens créés entre les particules. Cette mesure peut être obtenue pour un volume donné par la différence entre le nombre de singulet présents en début de méthode (N1) et mesuré dans un volume (V) donné, et le nombre (N2) d'agrégats et de singulet encore présents en fin de réaction d'agrégation, mesuré dans le même volume (V) en fin de méthode. Cette différence (N1-N2) correspond au nombre de liens biologiques formés entre 2 particules fonctionnalisées pendant la mesure. Dans le cas où N1 comprendrait d'éventuels agrégats non spécifiques (agrégats constitués d'au moins 2 particules fonctionnalisées liées entre elles par tout autre moyen que par un objet d'intérêt) la mesure par différence selon l'invention permet de s'en affranchir.

Selon l'invention le signal utile est le taux d'agrégation (TA) qui correspond au nombre de liens biologiques formés, (N1-N2), rapporté au nombre total de singulets (N1), présents en début de mesure dans le milieu liquide comprenant ledit objet d'intérêt à doser, autrement dit après introduction et avant agrégation. Ainsi selon l'invention le taux d'agrégation s'exprime par TA = (N1-N2)/N1.

Ainsi, les mesures sont normalisées par rapport à l'état initial du système, ce qui pourra contribuer à diminuer l'effet d'éventuelles erreurs de préparation du mélange réactionnel.

Selon l'invention, la concentration en objet d'intérêt d'un échantillon inconnu peut être déterminée par report du taux d'agrégation mesuré sur une courbe d'étalonnage préalablement établie en mesurant le taux d'agrégation desdites particules fonctionnalisées en présence de quantités connues de l'objet d'intérêt.

On comprend de ce qui précède que la nouvelle méthode de quantification selon l'invention nécessite la détermination d'une part du nombre de singulets présents dans le milieu réactionnel en début de réaction (N1) et d'autre part du nombre total d'agrégats formés dans le milieu réactionnel et de singulets encore présents en fin de réaction d'agrégation (N2).

Les nombres (N1) ou (N2) peuvent être mesurés de la manière suivante : chaque particule ou agrégat peut être soumis à une détection par une méthode optique, électrique ou autre. Ainsi, le passage de chaque particule ou agrégat au niveau du détecteur donne naissance à une impulsion qui peut être enregistrée. Le nombre total d'impulsions durant la durée de comptage peut alors être déterminé. Avant l'étape d'agrégation, on mesure un nombre N1 de singulets. Après l'étape d'agrégation, on mesure un nombre N2 d'agrégats et de singulets restants. Selon l'invention, chaque agrégat formé, quelle que soit sa taille, comportant k particules agglutinées par k-1 liens, est compté comme un unique élément.

En outre la méthode selon l'invention permet de s'affranchir d'une séparation des populations sur une représentation mono ou multiparamétrique.

La méthode selon l'invention permet la mesure du nombre de liens tout en évitant de s'appuyer sur la variation d'une ou plusieurs grandeurs physiques des objets détectés ; l'intérêt de ne pas s'appuyer sur la variation d'une grandeur physique est qu'un petit agrégat pourrait présenter une amplitude inférieure à une grosse particule, menant à de mauvais classement des objets étudiés.

La méthode selon l'invention permet de prendre en compte tous les agrégats tels que définis précédemment de telle sorte que même aux fortes concentrations en objets d'intérêt, chaque agrégat formé sera considéré individuellement. Ainsi tous les liens créés par les objets d'intérêt seront pris en compte et la précision de la mesure en est améliorée, particulièrement en cas de concentration élevée en objets d'intérêt.

Un autre avantage de la méthode selon l'invention réside dans le fait qu'un choix de particules plus important s'offre pour conduire la réaction. En particulier, des particules qui présentent des propriétés intéressantes, comme le caractère superparamagnétique, pourront être utilisées pour favoriser la réaction, alors que les techniques de détection existantes empêchent leur utilisation, à cause de leur dispersion de taille.

Ainsi l'invention a pour objet premier une méthode de quantification dans un milieu liquide d'au moins un objet d'intérêt, caractérisée en ce qu'elle met en oeuvre des particules fonctionnalisées en surface par au moins un récepteur spécifique dudit objet d'intérêt à doser et dans laquelle dans :
- une première étape on met en contact lesdites particules fonctionnalisées avec l'objet d'intérêt à doser, on mélange pendant un temps donné (t₁) et on prélève immédiatement un volume (v) dudit mélange obtenu dans lequel on compte le nombre N1 de particules non agrégées (singulets) par une mesure en flux ;
- une seconde étape on incube ledit mélange obtenu à l'étape 1 pendant un temps (t₂) suffisant pour permettre la formation d'agrégats et on prélève un volume (v) et on compte le nombre N2 correspondant à la fois aux particules non agrégées et aux agrégats contenus dans le volume (v) après réaction par la même technique de mesure en flux que celle utilisée à l'étape 1 ;
- une troisième étape on détermine le taux d'agrégation TA = (N1-N2)/N1 (TA calculé) et on quantifie ledit objet d'intérêt par comparaison du TA calculé avec une gamme étalon (TA = f([C]) réalisée préalablement par la mesure du taux d'agrégation obtenu par la même technique de mesure en flux avec le même objet d'intérêt à des concentrations ([C]) dudit objet d'intérêt prédéterminées.

A titre d'exemple, l'objet d'intérêt à doser peut être contenu dans un milieu tel que les fluides biologiques, parmi lesquels les fluides corporels comme par exemple le sang, le sérum, le plasma, la salive, l'urine, le liquide céphalo-rachidien ou encore des extraits tissulaires comme la moelle osseuse. On peut aussi citer par exemple les rejets de stations d'épuration, les eaux destinées à la consommation, etc. Avantageusement l'invention s'adresse aux fluides biologiques ou aux extraits tissulaires.

Les collisions entre les particules fonctionnalisées sont dues au mouvement brownien naturel. Celui-ci dépend de plusieurs paramètres qui influent naturellement sur la fréquence des collisions. Il est possible de jouer sur ces paramètres pour créer des conditions favorables pour avoir un temps suffisant avant que les premières collisions n'aient lieu pendant lequel il est possible d'analyser le milieu avant que ladite réaction d'agrégation n'intervienne, pour par exemple mesurer la quantité de singulet présents en début de réaction (N1).

Par ailleurs, il est connu des techniques permettant d'augmenter la fréquence des collisions entre particules et ainsi accélérer considérablement la réaction d'agrégation. La méthode selon l'invention peut inclure ou non une telle étape d'accélération de la réaction d'agrégation par application au cours de la méthode d'une technique connue d'augmentation de la fréquence des collisions entre particules.

Ainsi, selon l'invention la méthode de quantification peut en outre comprendre une étape permettant d'augmenter la fréquence des collisions entre particules, ladite étape pouvant se placer entre la fin de la première étape et le début de la seconde ou encore être intégrée à la seconde étape.

Par particules fonctionnalisées selon l'invention, il est entendu tout type de particules fonctionnalisées décrites dans l'art antérieur et utilisable selon l'invention. A titre d'exemple on peut citer des billes métalliques ou en matière plastique comme par exemple en polystyrène, ou des particules de silice ou en polymère ou encore des particules des composition mixte (particules mixtes) comme par exemple des particules d'oxyde de fer recouvertes de plastique, ou encore des particules non solides comme par exemple des liposomes. Préférentiellement selon l'invention on pourra utiliser des particules mixtes.

Selon l'invention, la taille desdites particules peut être comprise entre 5 et 10000nm, et plus préférentiellement entre 100 et 1000nm.

Selon une forme de l'invention, lesdites particules pourront être sensibles ou avoir été rendues sensibles aux techniques utilisables pour augmenter la fréquence des collisions entre particules comme les techniques utilisant des champs magnétiques ou électriques ou des ultrasons. Préférentiellement on utilisera selon l'invention des particules magnétiques.

Au titre de particules magnétiques, on pourra utiliser selon l'invention des particules paramagnétiques, diamagnétiques, ferromagnétiques ou ferrimagnétiques ou encore superparamagnétiques. L'utilisation de telles particules permet d'accélérer l'étape d'agrégation, et de générer un signal plus important. Lorsqu'elles sont soumises à un champ magnétique, ces particules s'organisent en chaînes. La concentration locale est augmentée, et la réaction accélérée.

Ainsi, des agrégats peuvent être formés avec des concentrations faibles en objets d'intérêt et en particules. Le nombre de liens biologiques formés est alors faible, du fait de la faible concentration des différentes espèces. Les méthodes de mesure en flux s'adaptent particulièrement bien à la mesure de quantités faibles de particules, et peuvent donc être avantageusement utilisées dans la méthode de quantification selon l'invention pour mesurer le taux d'agrégation de particules magnétiques.

L'homme du métier saura sans difficulté choisir les particules adaptées à la mesure qu'il souhaite réaliser parmi les nombreux fournisseurs connus. Avantageusement selon l'invention, on peut utiliser des particules superparamagnétiques telles que sous un champ magnétique adéquat, elles conservent une capacité à tourner sur elles-mêmes sous l'effet du mouvement brownien tout en formant des chaînes.

Dès lors, toutes méthodes de mesure en flux connues peuvent être utilisées selon l'invention comme par exemple la cytométrie de flux ou encore l'électrophorèse capillaire ou l'écoulement dans un canal microfluidique. Avantageusement selon l'invention on pourra utiliser la cytométrie de flux.

Selon l'invention, ledit récepteur spécifique peut être, naturel ou synthétique, comme par exemple un peptide, une protéine, un acide nucléique, un saccharide, un lipide, une hormone, et toute autre substance biologique ou synthétique pour peu que ledit récepteur soit apte à se lier avec l'objet d'intérêt (Antibodies a laboratory manual E. Harlow et D. Lane, Cold Spring Harbor Laboratory, 1988).

L'homme du métier saura sans difficulté greffer si nécessaire des récepteurs sur des particules.

Ces récepteurs peuvent être immobilisés à la surface des particules par différentes techniques connues de l'homme du métier, comme par exemple par des interactions d'adsorption, covalentes et/ou de hautes affinités. A toutes fins utiles on pourra se référer à l'ouvrage "Bioconjugate Techniques", de G.T. Hermanson, (Academic Press, 1996)

La méthode selon l'invention utilise des conditions de réaction connues de l'homme du métier qu'il n'aura donc aucune difficulté à mettre en oeuvre.

Selon l'invention, il est possible d'ajouter au mélange réactionnel de la première étape un détergent non-ionique (Tween® 80), ionique (cholate de sodium, taurocholate de sodium) ou encore zwitterionique (3-[(3-Cholamidopropyl)dimethylammonio]-1-propanesulfonate (CHAPS)), une solution de protéines (Sérumalbumine bovine, Bovine SerumAlbumin, (BSA)), ou des polymères (Alcool polyvinilyque, PolyVinyl Alcohol (PVA)).

L'ajout d'un détergent permet d'éviter que deux particules restent liées après une collision sans pour autant qu'un objet d'intérêt ne soit impliqué. On évite ainsi les agrégations non spécifiques.

Selon l'invention, la durée de la première étape est courte et ne dure approximativement que le temps t1 d'un rapide mélange des particules dans le milieu. Ainsi la première étape peut durer de quelques secondes à quelques minutes, avantageusement au maximum 3 minutes, très avantageusement 2 minutes.

Cette première étape de la méthode, selon l'invention, permet de mesurer le nombre N1 de particules fonctionnalisées non agrégées dans le volume (v) prélevé en début de réaction. Pour cela une fraction du mélange réactionnel peut être prélevée dès l'introduction des particules dans le volume (v) prélevé contenant l'objet d'intérêt à doser et analysée. Le temps écoulé entre l'introduction des particules et le prélèvement d'une aliquote dudit mélange étant court devant le temps d'agrégation passive des particules dans le milieu liquide, cette valeur N1 correspond à la concentration en particules fonctionnalisées non agrégées introduites dans ledit milieu liquide en début de réaction.

Selon l'invention, la durée de la seconde étape peut être comprise entre 5 secondes et 3 heures, préférentiellement entre 5 et 60 minutes Ce temps peut permettre la formation du maximum d'agrégats de toutes tailles mais doit rester compatible avec une durée totale de la mesure raisonnable.

Selon une variante de l'invention, juste avant la seconde étape de la méthode ou pendant la seconde étape, il peut être appliqué un champ magnétique, un champ électrique ou des ultrasons afin d'augmenter la fréquence des collisions entre particules.

Par exemple, si on applique un champ magnétique le milieu peut subir de 1 à 10 cycles de champ de 3mT à 100mT, chaque cycle pouvant avoir une durée de 1 à 600 secondes, préférentiellement de 100 à 500 secondes, avantageusement 300 secondes en alternance avec des périodes de relaxation (pas de champ appliqué).

Selon l'invention ,à la troisième étape de la méthode, on calcule le taux d'agrégation TA = (N1-N2)/N1 (TA calculé). La concentration en objet d'intérêt dans l'échantillon peut alors être déterminée par comparaison du TA calculé avec une gamme étalon de TA préalablement établie en mesurant l'agrégation des particules en présence de quantités connues du même objet d'intérêt.

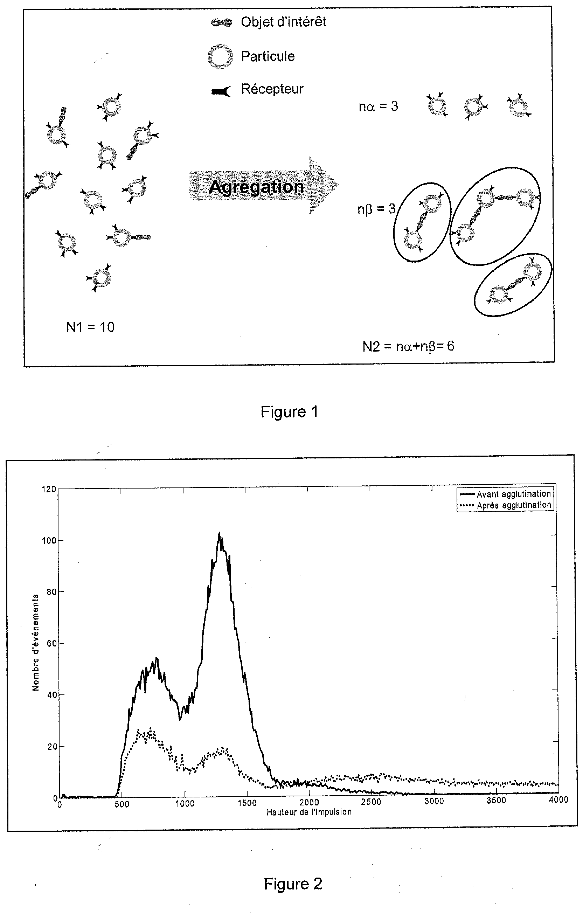

La figure 1 est une représentation schématique du principe de la méthode selon l'invention avec sur la partie gauche du schéma une représentation des particules fonctionnalisées libres de tout lien et les objets d'intérêt liés à une seule particule fonctionnalisée (singulets). C'est le nombre N1 de singulets qui est mesuré dans la première étape de la méthode selon l'invention. Sur la partie droite du schéma sont représentées d'une part les singulets encore présents après agrégation (nα) ou les particules fonctionnalisées impliquées dans des agrégats (nβ), la somme nα+nβ correspondant au nombre N2 de singulets et d'agrégats après agrégation. On comprend alors que N1-N2 (10-6=4) correspond au nombre de liens dans les nβ (3) agrégats formés et que dans ce cas théorique le Taux d'agrégation (TA) est égal à (N1-N2)/N1 = (10-6)/10 = 0,4.
La figure 2 montre les résultats de la détection par l'automate pour une agrégation en présence de 1pM de CRP. La courbe en trait continue représente les impulsions détectées pendant 30 secondes par l'automate de mesure avant agrégation. La somme de ces impulsions correspond à N1. La courbe en trait pointillé représente les impulsions détectées pendant 30 secondes par l'automate de mesure après agrégation. La somme de ces impulsions correspond à N2.
La figure 3 présente la courbe étalon obtenue par mesure du taux d'agrégation de particules de 500nm pour différentes concentrations en CRP (0 à 5pM).
La figure 4 présente la courbe étalon obtenue par mesure du taux d'agrégation de particules de 200nm pour différentes concentrations en CRP (0 à 15pM).
La figure 5 présente la courbe étalon obtenue par mesure du taux d'agrégation de particules de 200nm pour différentes concentrations en Sérumalbumine bovine biotynilée (0 à 500pM).

D'autres objets, caractéristiques et avantages de l'invention pourront ressortir des exemples qui suivent.

### Exemple 1 : Dosage de la CRP (Protéine C-réactive, C-Reactive Protein) en utilisant des particules superparamagnétiques de 500nm de diamètre

Des anticorps polyclonaux anti-CRP (L66616G, Meridian Life Science) (environ 10 µg d'anticorps par mg de billes) ont été greffées sur des particules superparamagnétiques de 500nm de diamètre (MasterBeads Carboxylic Acid 0215, Ademtech).

Les essais ont été conduits dans un tampon glycine 30mM, pH 8.5 contenant des concentrations variables en CRP (ABX Pentra CRP cal, Horiba Medical).

Pour limiter la formation de liens entre particules en l'absence de CRP, de l'acide taurocholique (T4009, Sigma-Aldrich) a été ajouté au milieu à une concentration finale de 3mM dans le mélange réactionnel.

La concentration finale en particules dans le milieu est d'environ 0,6pM. L'analyse en flux est effectuée en illuminant les particules avec un laser travaillant à 488nm. La diffusion à 90° est mesurée pour chaque objet traversant la cellule de mesure par un photomultiplicateur de marque Hamamatsu, modèle H9307-02.

Après mise en contact des différents réactifs, le milieu est laissé à incuber durant 2min. Pendant ces 2min, un volume (V1) de 53µL du mélange est prélevé et injecté dans l'analyseur en flux. Celui-ci assure une dilution au 1/100 du volume V1 et effectue un comptage durant 30s sur un volume V2 de 35,5µL de ce mélange. Le nombre singulets présents dans la suspension en début de réaction (N1) dans ledit volume V2 est alors déterminé.

Après 2min, le milieu subit 5 cycles de champ, composés de 30s sous 10mT, puis 300s sous 3mT et enfin 30s de relaxation sous 0mT.

Après le cycle d'aimantation, un second volume (V1) du mélange, identique au volume prélevé précédemment (53µl), est prélevé et analysé par l'analyseur en flux en suivant le même cycle de préparation. Cette mesure permet de déterminer le nombre d'agrégats formés (N2).

La figure 2 montre les résultats de la détection par l'automate pour une agrégation en présence de 1pM de CRP. Elle représente le nombre d'éléments détectés dans le volume (V2) pendant 30 secondes en fonction de la hauteur de l'impulsion qui leur est associée, elle-même fonction de la taille de l'objet. On constate qu'avant agrégation (trait continu), la suspension présente une distribution complexe avec de nombreuses tailles de particules présentes. Après agrégation (trait en pointillé), il n'apparaît pas de population clairement associée aux agrégats de particules, cependant le nombre total d'objets détectés (aire sous la courbe) a manifestement diminué.

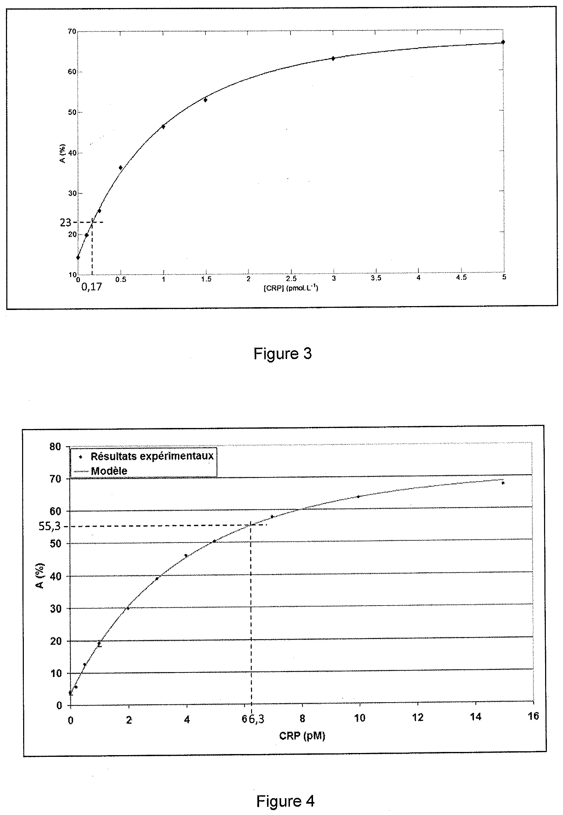

Etablissement de la courbe étalon :
En suivant le protocole décrit précédemment, le taux d'agrégation a été déterminé pour différentes concentrations en CRP (de 0 à 5pM). Les résultats sont présentés sur la figure 3.

On constate que le taux d'agrégation varie effectivement avec la concentration en CRP dans le milieu. A partir de l'écart-type mesuré sur plusieurs répétitions en l'absence de CRP, la limite de détection de ce système a pu être évaluée à 25fM de CRP, pour une durée de l'analyse de 35min environ.

Dosage de la concentration en CRP dans une solution inconnue :
Un échantillon de sérum, de concentration en CRP inconnue, a été dilué 10000 fois dans un tampon glycine 30mM, pH8.5. 27,6µL de ce mélange ont été ajoutés à un tampon glycine 30mM contenant les mêmes particules superparamagnétiques de 500nm de diamètre (MasterBeads Carboxylic Acid 0215, Ademtech) fonctionnalisées qu'utilisées précédemment ainsi que de l'acide taurocholique. Le volume final du milieu est de 600µL, avec une concentration finale en particules de 0,6pM et en acide taurocholique de 3mM.

Après mise en contact des différents réactifs, le milieu est laissé à incuber durant 2min. Pendant ces 2min, un volume (V1) de 53µL du mélange est prélevé et injecté dans l'analyseur en flux. Celui-ci assure une dilution au 1/100 du volume V1 et effectue un comptage durant 30s sur un volume (V2) de 35,5µL de ce mélange. Le nombre singulets présents dans la suspension en début de réaction (N1) dans ledit volume V2 est alors déterminé.

Après 2 minutes, le milieu subit 5 cycles de champ, composés de 30s sous 10mT, 300s sous 3mT et 30s de relaxation sous 0mT.

Après le cycle d'aimantation, un second volume (V1) du mélange, identique au volume prélevé précédemment (53µl), est prélevé et analysé de manière identique au premier prélèvement afin de déterminer la valeur de N2.

En suivant le protocole décrit précédemment, il a ainsi été possible de déterminer pour l'échantillon inconnu un taux d'agglutination TA=0,23. Le report sur la courbe d'étalonnage permet de déterminer une concentration en CRP dans le milieu réactionnel égale à 0,17pM, soit une concentration en CRP dans le sérum inconnu de 37nM.

### Exemple 2: Dosage de la CRP en utilisant des particules superparamagnétiques de 200nm.

Etablissement de la courbe étalon :
Environ 35 µg d'anticorps polyclonaux anti-CRP (L66616G, Meridian Life Science) par mg de particules ont été greffées sur des particules magnétiques de diamètre 200nm (Carboxyl Adembeads, 0212, Ademtech).

Les essais ont été conduits dans un tampon glycine 30mM, pH 8.5 contenant des concentrations variables en CRP (ABX Pentra CRP cal, Horiba Medical).

Pour limiter la formation de liens entre particules en l'absence de CRP, de la saponine (30502-42, Nacalai Tesque) a été ajoutée au milieu à 0,08% en poids dans le mélange réactionnel comme détergent.

La concentration finale en particules dans le milieu est d'environ 3pM.

Après mise en contact des différents réactifs, le milieu est laissé à incuber durant 2min. Pendant ces 2minutes, un volume (V1) de 53µL du mélange est prélevé et injecté dans l'analyseur en flux. Celui-ci assure une dilution au 1/1200 du volume V1 et effectue un comptage durant 30s sur un volume (V2) de 35,5µL de ce mélange. Le nombre de singulets présents dans la suspension en début de réaction (N1) dans ledit volume V2 est alors déterminé.

Après 2 minutes, le milieu subit 2 cycles de champ magnétique, composés de 30 s sous 50mT, 300 s sous 20mT et 30 s de relaxation sous 0mT.

Après le cycle d'aimantation, un second volume (V1) du mélange, identique au volume prélevé précédemment (53µl), est prélevé et analysé de manière identique au premier prélèvement afin de déterminer la valeur de N2. Cette mesure permet de déterminer le nombre d'agrégats formés (N2).

En suivant le protocole décrit précédemment, le taux d'agrégation a été déterminé pour différentes concentrations en CRP (de 0 à 16pM). La courbe étalon obtenue est présentée sur la figure 4.

A partir des répétitions en l'absence de CRP, la limite de détection a été évaluée à 100fM de CRP, pour une durée de l'analyse de 15min environ.

Dosage de la concentration en CRP dans une solution inconnue :
Un échantillon de sérum, de concentration en CRP inconnue, a été dilué 100 fois dans un tampon glycine 30mM, pH 8.5. 2µL de ce mélange ont été ajoutés à un tampon glycine 30mM contenant les mêmes particules magnétiques de 200nm de diamètre fonctionnalisées ainsi que de la saponine. Le volume final du milieu est de 600µL, avec une concentration finale en particules de 3pM et en saponine de 0,08% (poids/volume).

Après mise en contact des différents réactifs, le milieu est laissé à incuber durant 2min. Pendant ces 2min, un volume (V1) de 53µl du mélange est prélevé et injecté dans l'analyseur en flux. Celui-ci assure une dilution au 1/1200 du volume V1 et effectue un comptage durant 30s sur un volume (V2) de 35,5µL de ce mélange. Le nombre de singulets présents dans la suspension en début de réaction (N1) dans ledit volume V2 est alors déterminé.

Après 2min, le milieu subit 2 cycles de champ, composés de 30s sous 50mT, 300s sous 20mT et 30s de relaxation sous 0mT.

Après le cycle d'aimantation, un second volume (V1) du mélange, identique au volume prélevé précédemment (53µl), est prélevé et analysé de manière identique au premier prélèvement afin de déterminer la valeur de N2. Cette mesure permet de déterminer le nombre d'agrégats formés (N2).

On a ainsi pu déterminer pour l'échantillon inconnu un taux d'agglutination TA=0,55. Le report sur la courbe d'étalonnage permet de déterminer une concentration en CRP dans le milieu réactionnel égale à 6,3pM, soit une concentration en CRP dans le sérum inconnu de 189nM.

### Exemple 3: Dosage de la biotine en utilisant des particules superparamagnétiques de 200nm

Etablissement de la courbe étalon :
Des particules de 200nm de diamètre recouvertes de streptavidine ont été utilisées (Bio-Adembeads Streptavidin 0312, Ademtech).

Les essais ont été conduits dans un tampon glycine 30mM, pH 8.5 contenant 0,5 % de Sérumalbumine bovine (BSA Protease Free, ID Bio) ainsi que des concentrations variables de Sérumalbumine bovine biotinylée (BSAb) (A8549, Sigma-Aldrich). La concentration finale en particules dans le milieu vaut 6pM environ.

Après mise en contact des différents réactifs, le milieu est laissé à incuber durant 2min. Pendant ces 2min, un volume (V1) de 53µL du mélange est prélevé et injecté dans l'analyseur en flux. Celui-ci assure une dilution au 1/2400 du volume V1 et effectue un comptage durant 30s sur un volume (V2) de 35,5µL de ce mélange. Le nombre de singulets présents dans la suspension en début de réaction (N1) dans ledit volume V2 est alors déterminé.

Après 2min, le milieu subit 2 cycles de champ, composés de 30 s sous 50mT, 300 s sous 20mT et 30s de relaxation sous 0mT.

Après le cycle d'aimantation, un second volume (V) du mélange, identique au volume prélevé précédemment (53µl), est prélevé et analysé de manière identique au premier prélèvement afin de déterminer la valeur de N2.Cette mesure permet de déterminer le nombre d'agrégats formés (N2).

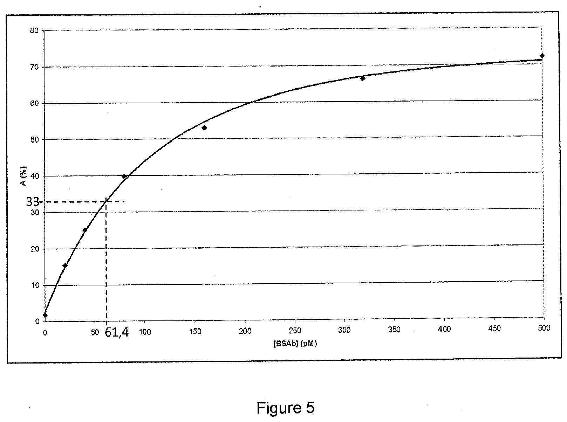

En suivant le protocole décrit précédemment, le taux d'agrégation a été déterminé pour différentes concentrations en biotine (de 0 à 500pM). La courbe étalon obtenue est présentée sur la figure 5. A partir des répétitions en l'absence de BSA biotinylée, la limite de détection a été évaluée. Les conditions expérimentales n'ont pas été optimisées pour cet essai, et on trouve une limite de détection élevée, de l'ordre de 7pM de BSA biotinylée, pour une durée de l'analyse de 15min environ.

Dosage de la concentration en biotine dans une solution inconnue :
Un échantillon de concentration en BSAb inconnue, a été dilué 10 fois dans une solution de BSA à 5%. 60µL de ce mélange ont été ajoutés à un tampon glycine 30mM contenant les mêmes particules magnétiques de 200nm de diamètre fonctionnalisées. Le volume final du milieu est 600µL, avec une concentration finale en particules de 6pM et en BSA de 0,5% (poids/volume).

Après mise en contact des différents réactifs, le milieu est laissé à incuber durant 2min. Pendant ces 2min, un volume (V1) de 53µl du mélange est prélevé et injecté dans l'analyseur en flux. Celui-ci assure une dilution au 1/2400è du volume V1 et effectue un comptage durant 30s sur un volume (V2) de 35,5µL de ce mélange. Le nombre de singulets présents dans la suspension en début de réaction (N1) dans ledit volume V2 est alors déterminé.

Après 2min, le milieu subit 2 cycles de champ, composés de 30s sous 50mT, 300s sous 20mT et 30s de relaxation sous 0mT.

Après le cycle d'aimantation, un second volume (V1) du mélange, identique au volume prélevé précédemment (53µl), est prélevé et analysé de manière identique au premier prélèvement afin de déterminer la valeur de N2. Cette mesure permet de déterminer le nombre d'agrégats formés (N2).

On a ainsi pu déterminer pour l'échantillon inconnu un taux d'agglutination TA=0,33. Le report sur la courbe d'étalonnage permet de déterminer une concentration en BSAb dans le milieu réactionnel égale à 61,4pM, soit une concentration en BSAb dans l'échantillon inconnu de 614pM.

## Revendications

1. Méthode de quantification dans un milieu liquide d'au moins un objet d'intérêt, **caractérisée en ce qu'**elle met en oeuvre des particules fonctionnalisées en surface par au moins un récepteur spécifique dudit objet d'intérêt à doser et dans laquelle dans :
• une première étape on met en contact lesdites particules fonctionnalisées avec l'objet d'intérêt à doser, on mélange pendant un temps donné (t₁) et on prélève immédiatement un volume (v) dudit mélange obtenu dans lequel on compte le nombre N1 de particules non agrégées (singulets) par une mesure en flux ;
• une seconde étape on incube ledit mélange obtenu à l'étape 1 pendant un temps (t₂) suffisant pour permettre la formation d'agrégats et on prélève un volume (v) et on compte le nombre N2 correspondant à la fois aux particules non agrégées et aux agrégats contenus dans le volume (v) après réaction par la même technique de mesure en flux que celle utilisée à l'étape 1 ;
• une troisième étape on détermine le taux d'agrégation TA = (N1-N2)/N1 (TA calculé) et on quantifie ledit objet d'intérêt par comparaison du TA calculé avec une gamme étalon (TA = f([C]) réalisée préalablement par la mesure du taux d'agrégation obtenu par la même technique de mesure en flux avec le même objet d'intérêt à des concentrations ([C]) dudit objet d'intérêt prédéterminées.

2. Méthode selon la revendication 1, **caractérisée en ce que** ledit objet d'intérêt à doser est, une protéine, un anticorps, un acide nucléique, une cellule, un fragment de cellule, un microorganisme, un fragment de microorganisme ou encore une molécule chimique.

3. Méthode selon l'une quelconque des revendications 1 ou 2, **caractérisée en ce que** l'objet d'intérêt à doser est contenu dans un fluide biologique ou un extrait tissulaire ou des rejets de stations d'épuration ou des eaux destinées à la consommation, avantageusement un fluide biologique ou un extrait tissulaire.

4. Méthode selon la revendication 3, **caractérisée en ce que** ledit fluide biologique est choisi parmi le sang, le sérum, le plasma, la salive, l'urine, le liquide céphalo-rachidien et ledit extrait tissulaire est de la moelle osseuse.

5. Méthode selon l'une quelconque des revendications 1 à 4, **caractérisée en ce que** ledit récepteur est choisi parmi les peptides, les protéines, les acides nucléiques, les saccharides, les lipides, les hormones.

6. Méthode selon l'une quelconque des revendications 1 à 5, **caractérisée en ce que** la taille desdites particules est comprise entre 5 et 10000nm, et plus préférentiellement entre 100 et 1000nm.

7. Méthode selon l'une quelconque des revendications 1 à 6, **caractérisée en ce que** les particules fonctionnalisées sont des particules magnétiques.

8. Méthode selon l'une quelconque des revendications 1 à 7, **caractérisée** en ce la durée de la première étape est de quelques secondes à quelques minutes, avantageusement au maximum 3 minutes, très avantageusement 2 minutes.

9. Méthode selon l'une quelconque des revendications 1 à 8, **caractérisée en ce qu'**à la seconde étape de la méthode on augmente la fréquence des collisions entre particules.

10. Méthode selon la revendication 9, **caractérisée en ce que** l'on augmente la fréquence des collisions entre particules par application d'un champ magnétique ou électrique ou application d'ultrasons.

11. Méthode selon l'une quelconque des revendications 1 à 10, **caractérisée en ce que** la durée de la seconde étape peut être comprise entre 5 secondes et 3 heures, préférentiellement entre 5 et 60 minutes.

12. Méthode selon l'une quelconque des revendications 1 à 11, **caractérisée en ce que** les mesures sont réalisées en flux.

13. Méthode selon la revendication 12, **caractérisée en ce que** les mesures sont réalisées par cytométrie de flux ou électrophorèse capillaire ou écoulement dans un canal microfluidique, préférentiellement par cytométrie de flux.

## Patentansprüche

1. Verfahren zum Quantifizieren von wenigstens einem Objekt von Interesse in einem flüssigen Medium, **dadurch gekennzeichnet, dass** es Partikel, die durch wenigstens einen für das genannte zu probierende Objekt von Interesse spezifischen Empfänger an der Oberfläche funktionalisiert sind, einsetzt, und bei dem:
• in einem ersten Schritt die genannten funktionalisierten Partikel mit dem zu probierenden Objekt von Interesse in Kontakt gebracht und für eine gegebene Zeit (t₁) gemischt werden und sofort ein Volumen (v) des genannten erhaltenen Gemischs genommen wird, in dem die Anzahl N1 von nicht aggregierten Partikeln (Singuletts) mittels Durchflussmessung gezählt wird;
• in einem zweiten Schritt das genannte, in Schritt 1 erhaltene Gemisch für eine Zeit (t₂) inkubiert wird, die ausreicht, um die Bildung von Aggregaten zuzulassen, und ein Volumen (v) genommen wird und die Anzahl N2 gezählt wird, die gleichzeitig den nicht aggregierten Partikeln und den Aggregaten in dem Volumen (v) nach der Reaktion mit derselben Durchflussmesstechnik wie in Schritt 1 entspricht;
• in einem dritten Schritt die Aggregationsrate AR = (N1-N2)/N1 (berechnete AR) bestimmt und das genannte Objekt von Interesse durch Vergleichen der berechneten AR mit einem Standardbereich (AR = f([C]) quantifiziert wird, zuvor realisiert durch die Messung der Aggregationsrate, erhalten mit derselben Durchflussmesstechnik mit demselben Objekt von Interesse bei vorbestimmten Konzentrationen ([C]) des genannten Objekts von Interesse.

2. Verfahren nach Anspruch 1, **dadurch gekennzeichnet, dass** das genannte zu probierende Objekt von Interesse ein Protein, ein Antikörper, eine Nukleinsäure, eine Zelle, ein Zellfragment, ein Mikroorganismus, ein Mikroorganismusfragment oder auch ein chemisches Molekül ist.

3. Verfahren nach Anspruch 1 oder 2, **dadurch gekennzeichnet, dass** das zu probierende Objekt von Interesse in einem biologischen Fluid oder einem Gewebeextrakt oder in Abwässern von Kläranlagen oder in Trinkwasser enthalten ist, vorteilhafterweise ein biologisches Fluid oder ein Gewebeextrakt.

4. Verfahren nach Anspruch 3, **dadurch gekennzeichnet, dass** das genannte biologische Fluid ausgewählt ist aus Blut, Serum, Plasma, Speichel, Urin, Gehirn-Rückenmarksflüssigkeit, und das genannte Gewebeextrakt Knochenmark ist.

5. Verfahren nach einem der Ansprüche 1 bis 4, **dadurch gekennzeichnet, dass** der genannte Empfänger ausgewählt ist aus Peptiden, Proteinen, Nukleinsäuren, Sacchariden, Lipiden, Hormonen.

6. Verfahren nach einem der Ansprüche 1 bis 5, **dadurch gekennzeichnet, dass** die Größe der genannten Partikel zwischen 5 und 10.000 nm, bevorzugter zwischen 100 und 1000 nm liegt.

7. Verfahren nach einem der Ansprüche 1 bis 6, **dadurch gekennzeichnet, dass** die funktionalisierten Partikel magnetische Partikel sind.

8. Verfahren nach einem der Ansprüche 1 bis 7, **dadurch gekennzeichnet, dass** die Dauer des ersten Schritts von mehreren Sekunden bis zu mehreren Minuten, vorteilhafterweise höchstens 3 Minuten, äußerst vorteilhafterweise 2 Minuten beträgt.

9. Verfahren nach einem der Ansprüche 1 bis 8, **dadurch gekennzeichnet, dass** im zweiten Schritt des Verfahrens die Häufigkeit von Kollisionen zwischen Partikeln zunimmt.

10. Verfahren nach Anspruch 9, **dadurch gekennzeichnet, dass** die Häufigkeit von Kollisionen zwischen Partikeln durch Anwenden eines magnetischen oder elektrischen Felds oder Anwenden von Ultraschall erhöht wird.

11. Verfahren nach einem der Ansprüche 1 bis 10, **dadurch gekennzeichnet, dass** die Dauer des zweiten Schritts zwischen 5 Sekunden und 3 Stunden, vorzugsweise zwischen 5 und 60 Minuten liegen kann.

12. Verfahren nach einem der Ansprüche 1 bis 11, **dadurch gekennzeichnet, dass** die Messungen im Durchfluss realisiert werden.

13. Verfahren nach Anspruch 12, **dadurch gekennzeichnet, dass** die Messungen durch Durchflusszytometrie oder Kapillarelektrophorese oder Strömung in einem mikrofluidischen Kanal, vorzugsweise durch Durchflusszytometrie realisiert werden.

## Claims

1. Method for quantifying, in a liquid medium, at least one object of interest, **characterized in that** it utilizes particles that are surface-functionalized by at least one receptor specific to said object of interest to be assayed and in which, in:
• a first step, said functionalized particles are brought into contact with the object of interest to be assayed, mixed for a given time (t₁) and a volume (v) of said obtained mixture is immediately collected, in which the number N1 of non-aggregated particles (singlets) is counted by means of a flow measurement;
• a second step, said mixture obtained in step 1 is incubated for a sufficient time (t₂) to allow the formation of aggregates and a volume (v) is collected and the number N2 corresponding both to the non-aggregated particles and to the aggregates contained in the volume (v) after reaction is counted by means of the same flow measurement technique as that used in step 1;
• a third step, the aggregation rate AR = (N1-N2)/N1 (calculated AR) is determined and said object of interest is quantified by comparing the calculated AR with a standard range (AR = f([C]) produced beforehand by measuring the aggregation rate obtained by means of the same flow measurement technique with the same object of interest at predetermined concentrations ([C]) of said object of interest.

2. Method according to claim 1, **characterized in that** said object of interest to be assayed is a protein, an antibody, a nucleic acid, a cell, a cell fragment, a microorganism, a microorganism fragment or also a chemical molecule.

3. Method according to any one of claims 1 or 2, **characterized in that** the object of interest to be assayed is contained in a biological fluid or a tissue extract or wastewater treatment plant effluents or water intended for consumption, advantageously a biological fluid or a tissue extract.

4. Method according to claim 3, **characterized in that** said biological fluid is selected from blood, serum, plasma, saliva, urine, cerebrospinal fluid and said tissue extract is bone marrow.

5. Method according to any one of claims 1 to 4, **characterized in that** said receptor is selected from peptides, proteins, nucleic acids, saccharides, lipids, hormones.

6. Method according to any one of claims 1 to 5, **characterized in that** the size of said particles is comprised between 5 and 10,000 nm, and more preferably between 100 and 1000 nm.

7. Method according to any one of claims 1 to 6, **characterized in that** the functionalized particles are magnetic particles.

8. Method according to any one of claims 1 to 7, **characterized in that** the duration of the first step is from a few seconds to a few minutes, advantageously a maximum of 3 minutes, very advantageously 2 minutes.

9. Method according to any one of claims 1 to 8, **characterized in that**, in the second step of the method, the frequency of the collisions between particles is increased.

10. Method according to claim 9, **characterized in that** the frequency of the collisions between particles is increased by the application of a magnetic or electric field or application of ultrasound.

11. Method according to any one of claims 1 to 10, **characterized in that** the duration of the second step can be comprised between 5 seconds and 3 hours, preferably between 5 and 60 minutes.

12. Method according to any one of claims 1 to 11, **characterized in that** the measurements are carried out in a flow.

13. Method according to claim 12, **characterized in that** the measurements are carried out by means of flow cytometry or capillary electrophoresis or flow in a microfluidic channel, preferably by means of flow cytometry.
